# EUROPÄISCHE PATENTANMELDUNG

(11) **EP 2 430 978 A1**
(43) Veröffentlichungstag der Anmeldung: **21.03.2012**
(21) Anmeldenummer: 11405298.8
(22) Anmeldetag: 09.08.2011
(51) Int. Cl.: A61B 5/16, A61B 5/18

(54) **Reflexmessgerät zur Bestimmung der Reaktionsfähigkeit eines Lenkers und dessen Anwendung**

(30) Priorität: 15.09.2010 CH 15002010
(71) Anmelder: Erbicol SA, 6828 Balerna (CH)
(72) Erfinder: Marcon, Giorgio, 31040 Giavera Del Montello (IT); Rubboli, Gian Mario, 20100 Milano (IT)
(74) Vertreter: Gaggini, Carlo

(57) **Zusammenfassung**

Die vorliegende Erfindung betrifft ein Reflexmessgerät zum Feststellen der Reaktionsfähigkeit eines Fahrzeuglenkers bzw. seiner Fahrtauglichkeit, ein Fahrzeug zu lenken, auch unter dem Einfluss von Alkohol, Drogen, Ermüdung, Stress oder normale Auswirkung des Alters.

Das Reflexmessgerät umfasst im Wesentlichen ein Fahrzeug-Lenkrad (1), das als Sensor zum Feststellen der Reaktionszeit des Fahrzeuglenkers dient, eine Vorrichtung, welche die Zeitspanne zwischen der Auslösung der Signalvorrichtung (2;3;4) und der Reaktion des Fahrzeuglenkers messen kann (beispielsweise ein analoger oder digitaler Chronograf (8)), einen Sessel (5) mit Sicherheitsgurte (6), welcher als Einschalt-Vorrichtung für das Reflexmessgerät dienen kann, sobald der Fahrzeuglenker auf dem Sessel (5) Platz nimmt.

Das Reflexmessgerät ist als Apparat vorgesehen, mittels welcher die Fahrtüchtigkeit eines Fahrzeuglenkers festgestellt werden kann, zur freiwilligen Verwendung ohne Zwang am Ausgang von Diskotheken, Bars, Nachtlokalen, Fahrschulen, Ladenpassagen, usw., oder zur Verwendung durch die Polizei zur zwangsweisen Durchführung von Kontrollen, die nicht bloss den Blutalkoholpegel des Lenkers - ein Messwert, der für die wirkliche Fahrtauglichkeit oft nicht massgebend ist - in Betracht ziehen, sondern auch der übrigen Faktoren, welche die Reaktionsfähigkeit beeinträchtigen.

## Beschreibung

Die vorliegende Erfindung betrifft ein Reflexmessgerät gemäss dem Oberbegriff des Anspruchs 1 sowie dessen Anwendung in der Praxis gemäss den Ansprüchen 11 und 12.

Das Problem der Bestimmung der Reaktionsfähigkeit eines Fahrzeuglenkers, also der Schnelligkeit und Sicherheit seiner Reaktion auf ein unvorhergesehenes Auftauchen einer Gefahr, stellt einen der wichtigsten Aspekte in der Prävention gegen Autounfälle dar. Unzählige Anstrengungen sind im Lauf der letzten Jahrzehnte unternommen worden, um die Zahl und Schwere der Unfälle zu vermindern, die sowohl Massnahmen an den Fahrzeugen selbst (wie beispielsweise Aufschlags-Dämpfungen, grössere Widerstandsfähigkeit des Fahrzeugs und somit kontrollierte Verformbarkeit im Fall von Zusammenstössen) betreffen, als auch Vorkehrungen zur Verbesserung der Reaktionsfähigkeit des Fahrzeuglenkers im Fall unvorhergesehener Vorfälle. Heute sind in den meisten Ländern strenge Gesetze in Kraft, die den Blutalkoholgehalt auf sehr tiefe Werte begrenzen, der mit verschiedenen Apparaten und Analysen gemessen werden kann. Bekannt ist jedoch, dass der Blutalkoholpegel lediglich einer der vielen Faktoren ist, welche die Reaktionsfähigkeit einer Person bestimmen. Müdigkeit, Drogenkonsum, Alter und andere Störungen psychischer Natur beeinflussen die wirkliche Fahrtüchtigkeit des Fahrzeuglenkers-die sich hauptsächlich in der Geschwindigkeit der Reaktionen auf unvorhergesehene Vorkommnisse äussert-stark. Der einfache Alkoholpegel-Test allein stellt daher keine sichere Bestimmungsart für die Fahrtüchtigkeit eines Autolenkers dar. Viel sicherer ist hingegen ein Test, der die Reaktionsfähigkeit des Fahrzeuglenkers misst, und dies ist der Zweck, der mittels des Reflexmessgerätes gemäss der vorliegenden Erfindung erfüllt werden soll. In vielen Ländern sind solche Tests bereits heute für ältere Personen gesetzlich vorgesehen, doch handelt es sich dabei um einfache Kontrollen der Sehschärfe und des Gehörs, was sich schwerlich als Reaktionsfähigkeit-Test betrachten lässt. Entscheidend in diesem Sinn war die Entwicklung eines Lenkrades für Fahrzeuge mit einer Einrichtung, die auf die Veränderungen des Druckes einer oder beider Hände des Lenkers reagiert, die das Lenkrad umgreifen. Als Basis dieses Prinzips ist hier die EP-B-1621442 mit den entsprechenden Äquivalenten in den wichtigsten Industrieländern zu nennen. Weitere Verbesserungen zu diesem Grundprinzip, das in der vorliegenden Erfindung angewendet wird, sind sodann jene, die in den Schweizer Patentanmeldungen Nr. CH/925/07 betreffend eine "Vorrichtung mit einem Sensor für hydraulischen oder pneumatischen Druck für die Lenkorgane", in Nr. CH/1179/07 betreffend ein "Verfahren zum Füllen des elastischen Schlauchs eines Drucksensors", und in CH/0867/08 betreffend ein "Verfahren zum Tarieren einer Vorrichtung mit flexiblem Schlauch" offengelegt sind. Alle diese genannten Erfindungen sind als integrierende Bestandteile der vorliegenden Patentanmeldung zu betrachten, da sie die wesentliche Basis bilden, auf der die erfindungsgemässe Lösung beruht.

Ziel der vorliegenden Erfindung ist daher, dem Lenker, der sich vorbereitet, sich den Gefahren der Strasse auszusetzen, die Möglichkeit zu geben, sich freiwillig einem Test zu unterziehen, der im Stande ist, seine wirkliche Fahrtauglichkeit festzustellen, nicht bloss in Abhängigkeit des Blutalkoholpegels, sondern aller psychophysischen Fähigkeiten wegen Müdigkeit, Drogenkonsum oder Medikamenteneinnahme, Stressniveau und Alter, usw., also insgesamt, seiner Fähigkeit richtig und schnell auf Umwelteinflüsse zu reagieren. Insbesondere ist dabei an Leute zu denken, die Diskotheken, Nachtlokale, Bars, usw., verlassen, welchen die Möglichkeit geboten werden soll, ihren Zustand ihrer Fahrtüchtigkeit freiwillig und ohne Zwang zu testen, bevor sie sich ans Steuer setzen. Erst wenn sie den Test bestanden haben, was normalerweise mit einem Zertifikats bestätigt wird, das ausgedruckt oder direkt auf ihr Mobiltelefon geschickt wird, weiss die Person, dass sie im Stande ist, mehr oder weniger gefahrlos zu fahren. Über eine geeignete Telefonverbindung kann das Testresultat auch jederzeit abgerufen werden, beispielsweise im Fall von Polizeikontrollen, um nachzuweisen, dass sich der Fahrzeuglenker zuvor dem Test unterzogen und diesen bestanden hatte, bevor er sich ans Steuer gesetzt hatte. Auch als obligatorische Prüfung für Senioren kann die Anwendung des Reflexmessgerätes eine bestgeeignete und vertrauenswürdige Darlegung der Fahrtüchtigkeit sicherstellen, dies auch bei Fahrschulen und anderen zuständigen Stellen.

Alle diese Zielsetzungen werden mit dem Reflexmessgerät erfüllt, das die Eigenschaften gemäss dem charakterisierenden Teil des Anspruchs 1, aufweist, allenfalls in Kombination mit den Eigenschaften der Ansprüche 2 bis 10.

Die Ansprüche 11 und 12 betreffen zwei bevorzugte Ausführungsformen des erfindungsgemässen Reflexmessgerätes.

Die vorliegende Erfindung ist im Folgenden näher beschrieben, unter Bezugnahme auf die Abbildungen. Diese zeigen in der
- Fig. 1: das erfindungsgemässe Reflexmessgerät von der Seite gesehen;
- Fig.2: das erfindungsgemässe Reflexmessgerät von hinten her gezeigt;
- Fig. 3: ein Detail des erfindungsgemässen Reflexmessgerätes, welches das Steuerrad zeigt, das mit einer auf Druckänderungen reagierenden Einrichtung versehen ist.

Bevor zur detaillierten Beschreibung der Abbildungen übergegangen wird, sind zwei generelle Aspekte der vorliegenden Erfindung zu präzisieren.

Erste Anmerkung: Das erfindungsgemässe Reflexmessgerät ist eine neuartige Kombination von Elementen, die gemäss dem Stand der Technik an sich alle bekannt sind. Die einzelnen Elemente können dabei Formen aufweisen, die sich von den hier darggestellten unterscheiden, ohne dass der Rahmen der Erfindung überschritten würde. Wichtig ist die Kombination ihrer Eigenschaften, dank der die angestrebten Zielsetzungen erreicht werden können, die sich je nach der vorgesehenen Anwendung des Reflexmessgerätes unterscheiden können (zum Beispiel: Reflexmessgerät für Diskotheken; Reflexmessgerät für Polizeikontrollen, Fahrschulen, usw.).

Zweite Anmerkung: Hinsichtlich des Steuerrades als immer erforderliches Element für die Bewertung der Reaktionsfähigkeit des Fahrzeuglenkers beruht die vorliegende Erfindung im Wesentlichen auf der in der in der EP-B-1621442 beschriebenen Lösung und deren aus den eingangs erwähnten Patentanmeldungen abgeleiteten Verbesserungen.

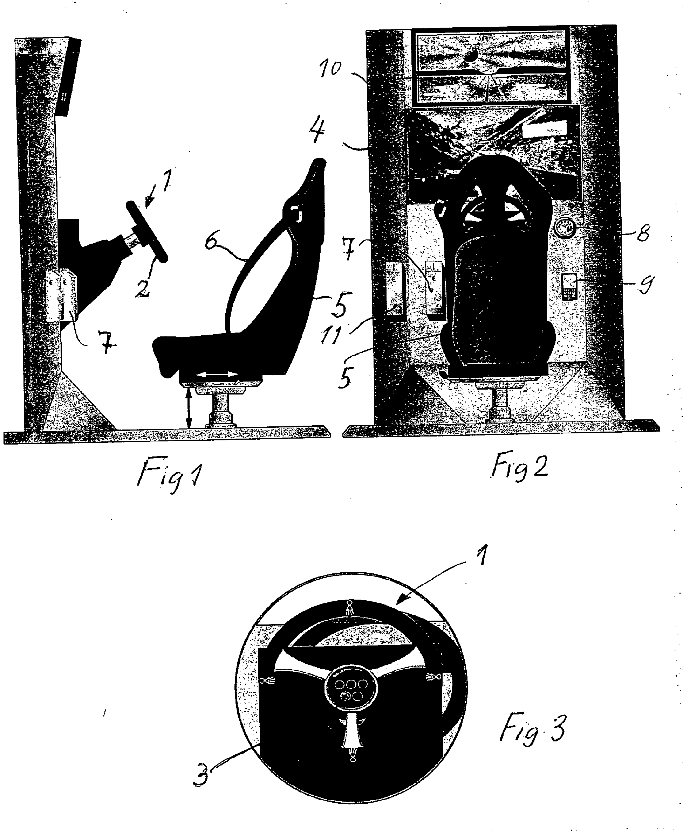

In den Figuren 1 bis 3 sind die gleichen Elemente mit den jeweils gleichen Bezugsziffern bezeichnet.

Mit 1 ist dabei ein Fahrzeug-Lenkrad bezeichnet, das mit einer Einrichtung versehen ist, die auf die Veränderungen des Druckes reagiert, der von einer oder beiden Händen des Fahrzeuglenkers ausgeübt wird, die den Kranz des Lenkrades umfassen. Die vorliegende Erfindung basiert auf dem Prinzip, dass das Mass der Reaktionsfähigkeit eines Fahrzeuglenkers, der korrekt steuert, indem er das Lenkrad mit einer oder vorzugsweise mit beiden Händen umfasst hält, auf bestmögliche Art ermittelt werden kann als zeitlicher Abstand vom Moment des Eintrittes der mittels einer Signaleinrichtung gezeigten Unfallgefahr (die das Einschalten einer Lampe 3 sein kann, oder besser - bei Verwendung eines Landschafts-Simulators - dem Aufscheinen eines Lichtes auf dem Bildschirm 4, eines unvermittelt auf der Fahrbahn auftauchenden Hindernisses, eines Tieres, eines Schlagloches, usw.) und der Reaktion des Fahrzeuglenkers. Erforderlich ist in jedem Fall eine Signaleinrichtung 2, 3, die verschieden ausgebildet sein kann, wie beispielsweise als Lämpchen 3, das zum Beispiel auf der Nabe des Lenkrades 1 (Siehe die Fig. 3) angebracht ist, oder als Bildschirm 4 eines Landschafts-Simulators wie in der Fig. 2 dargestellt. Auch akustische Signale (mit den optischen kombiniert oder nicht) können natürlich in Betracht gezogen werden.

Für das Funktionieren des erfindungsgemässen Reflexmessgerätes ist sodann eine beispielsweise elektronische Einrichtung zum Anzeigen der Reaktionszeiten erforderlich, die den zeitlichen Abstand zwischen dem Auslösen des Signals der Signaleinrichtung 2, 3, und der Reaktion der Einrichtung, die auf die Veränderungen des Druckes reagieren, den die Hand oder der Hände des Lenkers ausüben, wie dies in der EP-B-1621442 vorgesehen und beschrieben ist. Gemäss einer bevorzugten Lösung gemäss der vorliegenden Erfindung ist diese Einrichtung ein analoger oder digitaler Chronograf 8, der Hundertstel- und/oder Tausendstel-Sekunden messen kann.

Das erfindungsgemässe Reflexmessgerät umfasst ferner einen Auto-Sessel 5, dessen Sitzposition zur Anpassung an die physischen Eigenschaften des Lenkers einstellbar ist, und der mit einem Sicherheitsgurt 6 sowie mit einer Vorrichtung (nicht dargestellt) ausgerüstet ist, die das Reflexmessgerät einschaltet, wenn der Lenker auf dem Sessel 5 Platz nimmt.

Diese Einschalt-Einrichtung kann verschieden ausgebildet sein, wobei die Erfahrung gezeigt hat, dass eine bevorzugte Ausführungsform darin besteht, dass die Einschaltvorrichtung mit der Einführung des Sicherheitsgurtes 6 in den Verschluss verbunden ist, oder als bevorzugte Variante, mit einer Vorrichtung (nicht dargestellt), die das Vorhandensein des Lenkers auf dem Sessel 5 feststellet, falls der Sessel 5 mit einer entsprechenden Vorrichtung ausgerüstet ist (wie sie heute übrigens in vielen Fahrzeugen verwendet wird, um die Funktion der Airbags sinnvoll zu kontrollieren).

Für bestimmte Erfordernisse (beispielsweise um dem Lenker ein Beleg-Dokument über den bestandenen Test auszugeben, das im Fall einer Blutalkoholprüfung, usw., einem Polizeibeamten vorgelegt werden kann) kann das Reflexmessgerät auch mit einem Drucker für die Testresultate (nicht dargestellt in der Abbildung) sowie, für ähnliche Zwecke wie die beschriebenen, mit einem Mobiltelefon-Anschluss 9 versehen sein, über welchen die Resultate automatisch übertragen werden können, oder Testresultate von jeder externen Telefonstation aus abgerufen werden können, so dass dem kontrollierenden Polizeibeamten das Bestehen des Tests bestätigt werden kann, falls der Lenker die ausgedruckten Daten nicht mit sich führt.

Immer unter Berücksichtigung der besonderen Erfordernisse im Zusammenhang mit der Anwendung des Reflexmessgerätes sieht eine besondere bevorzugte Ausführungsform des erfindungsgemässen Reflexmessgerätes vor, dass dieses einen Münzautomaten 7 umfasst, an welchem der Test des Lenkers bezahlt werden kann. Diese Ausführungsform ist klarerweise besonders für Fälle vorgesehen, in welchen das Gerät, gemäss einer bevorzugten Art der Anwendung des Reflexmessgerätes, sich für die Verwendung in Diskotheken, Bars, Nachtlokalen, usw., eignen soll, selbstverständlich auf freiwilliger Basis ohne Zwang, um die Fahrtüchtigkeit des Kunden, der das Lokal verlässt, präventiv festzustellen. Selbstverständlich hat der Gastwirt in diesem Fall Anrecht auf eine entsprechende Entschädigung.

Zu unterstreichen ist, dass gemäss einer anderen bevorzugten Art der Anwendung das erfindungsgemässe Reflexmessgerät ein für die Polizei (beispielsweise die Strassenpolizei) bestens geeignetes Instrument sein kann, um die Eignung des Lenkers zum Fahren nicht nur in Abhängigkeit der Blutalkoholmesswert sondern auch infolge Ermüdung (die auch durch das fortgeschrittene Alter des Prüflings, Drogenkonsum und ähnliche Situationen wie beispielsweise Stress hervorgerufen sein kann), welche die Reaktionsfähigkeit eines Fahrzeuglenkers beeinflussen können, festzustellen und alles auf einem Datenträger (SD) in der "Black Box" registrieren zu können, welche die Europäischen Behörden in Zukunft verlangen werden.

Somit kann sichergestellt werden, dass das erfindungsgemässe Reflexmessgerät, das in seiner Grundstruktur zu einem eigentlichen Simulator für das Lenken eines Fahrzeugs auf der Strasse gemacht werden kann, in seinen verschiedenen Ausführungsformen, einen wichtige Beitrag für die Sicherheit im Strassenverkehr leisten kann und somit helfen kann, die Zahl der Unfälle - und der Todesopfer - wesentlich zu senken, die durch das Lenken von Fahrzeugen durch Personen in beeinträchtigtem Zustand oder die, einfacher ausgedrückt, fahruntüchtig sind, verursacht werden

### Liste der in den Figuren verwendeten Bezugsziffern

- 1: Lenkrad, ausgerüstet mit Vorrichtung gemäss EP-B-1621442
- 2: Lenkrad-Kranz
- 3: Lämpchen
- 4: Bildschirm
- 5: Sessel
- 6: Sicherheitsgurt
- 7: Münzautomat
- 8: Chronometer
- 9: Mobiltelefon
- 10: Projektor
- 11: zweiter Münzautomat zu Getränke-Automat für alkoholfreie Getränke

## Patentansprüche

1. Reflexmessgerät zum Feststellen der Reaktionsfähigkeit eines Fahrzeuglenkers bzw. seiner Fahrtauglichkeit mittels eines Tests, welches die folgenden Elemente umfasst:
- ein Lenkrad (1) eines Fahrzeugs mit einer Vorrichtung, die auf Veränderungen des Druckes einer oder beider Hände des Fahrzeuglenkers, die den Kranz (2) des Lenkrades (1) umfassen reagiert;
- eine Signal-Vorrichtung (2; 3; 4) zum Auslösen der Reaktion des Fahrzeuglenkers;
- eine Vorrichtung (8) zum Feststellen des zeitlichen Abstandes zwischen der Auslösung der Signalvorrichtung (2; 3; 4) und der Reaktion der Vorrichtung, die auf die Veränderungen des Druckes der Hand oder der Hände des Fahrzeuglenkers (Mass der Reaktionsfähigkeit des Fahrzeuglenkers) reagiert;
- ein Autosessel (5), dessen Sitzposition eingestellt werden kann, ausgerüstet mit einem Sicherheitsgurt (6);
- eine Einschaltvorrichtung (6) für das Reflexmessgerät, die anspricht, wenn sich der Fahrzeuglenker auf den Sessel (5) setzt.

2. Reflexmessgerät gemäss dem Anspruch 1,
**dadurch gekennzeichnet, dass**
die Signalvorrichtung (2; 3) aus einem Lämpchen (3) besteht, das in unvorhergesehenem Moment aufleuchtet und die Reaktion des Fahrzeuglenkers verlangt.

3. Reflexmessgerät gemäss dem Anspruch 1,
**dadurch gekennzeichnet, dass**
die Signalvorrichtung (2; 3) einen Bildschirm (4) eines Landschafts-Simulators umfasst, auf dem ein unerwartetes Hindernis auftaucht, das die Reaktion des Fahrzeuglenkers verlangt.

4. Reflexmessgerät gemäss dem Anspruch 1,
**dadurch gekennzeichnet, dass**
die Vorrichtung zum Messen des zeitlichen Abstandes ein analoger oder digitaler Chronograf (8) ist, der Hundertstel- und/oder Tausendstel-Sekunden messen kann.

5. Reflexmessgerät gemäss dem Anspruch 1,
**dadurch gekennzeichnet, dass**
die Vorrichtung zum Einschalten mit dem Verschluss des Sicherheitsgurtes (6 ) verbunden ist.

6. Reflexmessgerät gemäss dem Anspruch 1,
**dadurch gekennzeichnet, dass**
die Vorrichtung zum Einschalten mit einer Vorrichtung verbunden ist, welche das Vorhandensein eines Fahrzeuglenkers auf dem Sessel (5) feststellen kann.

7. Reflexmessgerät gemäss dem Anspruch 1,
**dadurch gekennzeichnet, dass**
es auch einen Drucker umfasst, der die Testresultate ausdruckt.

8. Reflexmessgerät gemäss dem Anspruch 1,
**dadurch gekennzeichnet, dass**
es auch eine Mobiltelefon-Verbindung (9) umfasst, mittels welcher die Testdaten übermittelt oder von irgendeiner externen Telefonstation aus abgerufen werden können.

9. Reflexmessgerät gemäss dem Anspruch 1,
**dadurch gekennzeichnet, dass**
es auch einen Münzautomaten (7) zur Bezahlung des Tests durch den Fahrzeuglenker umfasst.

10. Reflexmessgerät gemäss dem Anspruch 1,
**dadurch gekennzeichnet, dass**
es auch einen Projektor (10) zum projizieren von Werbebotschaften und/oder Video-Spielen umfasst.

11. Reflexmessgerät gemäss einem der Ansprüche 1 bis 10,
**dadurch gekennzeichnet, dass**
seine Anwendung insbesondere auf Diskotheken, Bars, Nachtlokale, Fahrschulen, Polizeistationen, usw., ausgelegt ist, um die Fahrtüchtigkeit des Fahrzeuglenkers/Kunden am Ausgang aus dem Lokal festzustellen.

12. Reflexmessgerät gemäss den Ansprüchen 1 bis 8,
**dadurch gekennzeichnet, dass**
seine Anwendung für die Polizei vorgesehen ist, um die Fahrtüchtigkeit des Lenkers festzustellen, in Abhängigkeit nicht nur vom Blutalkoholpegelsondern auch von der Ermüdung, von Drogenkonsum und ähnlichen anderen Situationen des Fahrzeuglenkers, welche die Reaktionsfähigkeit eines Fahrzeuglenkers beeinflussen.

13. Reflexmessgerät gemäss dem Anspruch 1,
**dadurch gekennzeichnet, dass**
es auch einen Dispenser für alkoholfreie Getränke umfasst.
